# FASCICULE DE BREVET EUROPEEN

(11) **EP 0 538 159 B1**
(45) Date de publication et mention de la délivrance du brevet: **17.04.1996**
(21) Numéro de dépôt: 92440025.2
(22) Date de dépôt: 20.02.1992
(51) Int. Cl.: A61L 2/18, A47K 5/12

(54) **Distributeur polyvalent de produits de désinfection placés à l'intérieur des contenants de forme variée**
Vielseitige Spender für in Behältern verschiedener Form enthaltene Desinfektionsmittel
Versatile dispenser for disinfectants held in containers of various shapes

(30) Priorité: 17.10.1991 FR 9113156
(43) Date de publication de la demande: 21.04.1993
(73) Titulaire: Laboratoires ANIOS S.A., F-59260 Lille-Hellemmes (Nord) (FR)
(72) Inventeur: Letartre, Thierry, F-59290 Wasquehal (Nord) (FR)
(74) Mandataire: Duthoit, Michel

(56) Documents cités:
- EP-A- 0 427 514
- DE-A- 3 842 959
- DE-A- 4 008 886
- DE-U- 9 103 349

## Description

La présente invention a pour objet un distributeur polyvalent destiné notamment à la distribution de produits de désinfection et/ou de nettoyage sous forme liquide, pulvérulente ou pâteuse, placés à l'intérieur de contenants de forme variée.

Elle trouvera son application dans le domaine de la désinfection et/ou du nettoyage notamment dans le domaine médical et chirurgical.

Dans le secteur médical et plus particulièrement dans celui de la chirurgie, il est impératif de respecter des règles d'hygiène strictes afin d'éviter les risques de contamination et/ou d'infections résultant de l'introduction d'agents pathogènes ou de microbes au cours des interventions chirurgicales.

C'est la raison pour laquelle le plus grand soin est apporté afin de rendre stérile l'ambiance des salles d'opération ainsi que les différents accessoires utilisés au cours de ces opérations.

Pour ce faire, on connaît différents dispositifs ou appareils dont la fonction est de permettre une désinfection ou une stérilisation des salles d'opération notamment par aspersion et/ou pulvérisation de substances antiseptiques.

Toutefois, il faut également que l'ensemble du personnel qui participe ces opérations porte des vêtements et présente des mains qui soient parfaitement propres.

Pour ce faire, le chirurgien et le personnel des salles d'opération se nettoient très soigneusement les mains et les avant-bras.

Ce lavage s'effectue généralement dans des dispositifs tels que des lavabos. Tous ces dispositifs sont reliés à une alimentation en eau associés à une filtration pour éviter que cette eau ne contienne des germes, des agents pathogènes ou, des microbes susceptibles de souiller les mains du personnel hospitalier.

Toutefois, malgré le plus grand soin apporté à la purification de l'eau, ce personnel médical doit utiliser des solutions désinfectantes qui sont placées dans un contenant disposé dans un distributeur à savon ou à produit désinfectant associé à ces dispositifs.

Ces distributeurs sont généralement adaptés pour recevoir des contenants d'une seule forme.

Le personnel chirurgical ou le chirurgien lui-même utilise souvent des produits distincts qui sont placés dans des contenants de formes différentes.

Pour répondre à cette exigence, soit l'on transverse le contenu de ces différents contenants à l'intérieur du contenant qui est placé dans le distributeur, soit l'on dispose ces différents contenants à la portée de mains du personnel médical.

On conçoit que, quelle que soit la solution envisagée, celle-ci n'est pas satisfaisante. C'est ainsi, qu'au cours du transvasement du liquide d'un contenant dans un autre, il se peut que le fond du contenant placé à l'intérieur du distributeur comporte encore une faible quantité de produit, ce qui conduit à un mélange de produits qui peuvent être incompatibles. Il en résulte alors une diminution de l'efficacité des agents actifs utilisés pour l'opération de lavage des mains.

De même, si l'on utilise simplement un contenant qui n'est pas disposé à l'intérieur du ditributeur, on accroît les risques d'introduction de microbes et d'agents pathogènes, ce qui est tout à fait contraire au but recherché.

La présente invention vise à pallier aux inconvénients des dispositifs actuellement utilisés en fournissant un distributeur polyvalent qui permet de recevoir des contenants de forme variée.

Un but de la présente invention est de fournir un distributeur qui est simple à utiliser et d'un faible coût de fabrication.

Un avantage de ce distributeur conforme à l'invention est également qu'il peut être facilement adapté à tous les dispositifs actuellement utilisés pour permettre le lavage des mains.

D'autres buts et avantages de la présente invention apparaîtront au cours de la description qui va suivre qui n'est cependant donnée qu'à titre indicatif.

A cet effet, le distributeur polyvalent destiné notamment à la distribution de produits de désinfection et/ou de nettoyage sous forme liquide, granuleuse ou pulvérulente placés à l'intérieur de contenants de forme variée est caractérisé par le fait qu'il comprend un boîtier qui présente une face avant ouverte destinée à recevoir au moins un contenant, et en ce qu'il comporte :
- des moyens pour faciliter la mise en place du contenant à l'intérieur du boîtier,
- des moyens pour maintenir le contenant dans une position déterminée à l'intérieur du boîtier en fonction de sa conformation,
- des moyens souples et flexibles pour distribuer le liquide depuis l'intérieur du contenant vers l'extérieur du distributeur.

La présente invention sera mieux comprise par la lecture de la description suivante accompagnée des dessins qui en font partie intégrante.

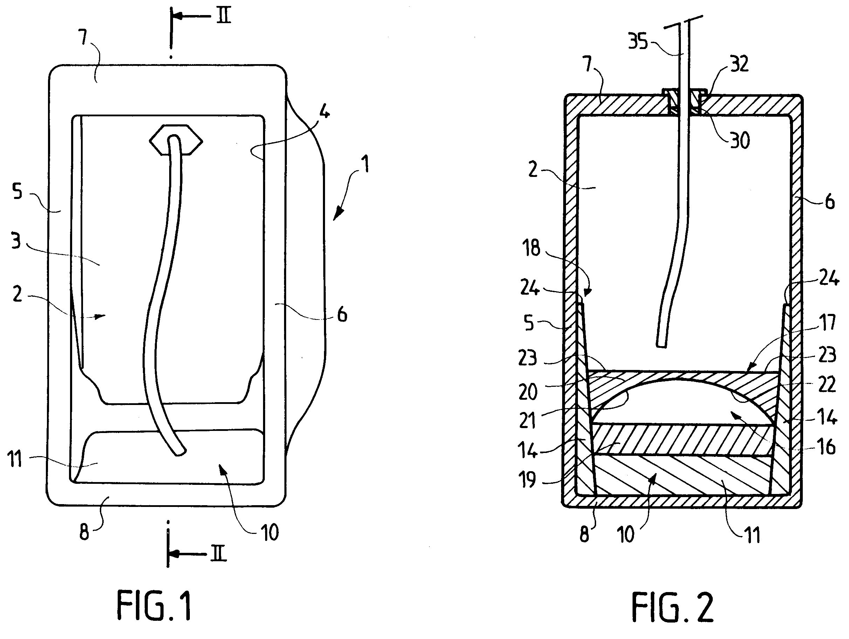

La figure 1 est une vue schématique qui représente un distributeur conforme à l'invention.

La figure 2 est une vue en coupe transversale selon la ligne II-II de la figure 1.

Les figures 3 à 5 sont des vues schématiques qui illustrent un distributeur à l'intérieur duquel sont placés différents types de contenants.

La figure 6 est une vue de face schématique qui illustre un contenant destiné à être placé à l'intérieur du distributeur.

La présente invention a pour objet un distributeur polyvalent de produits de désinfection et de nettoyage. Elle trouvera son application dans tous les domaines où il est nécessaire de se nettoyer les mains et les membres. Dans le mode de réalisation ci-dessus, on décrira un distributeur qui est plus particulièrement adapté pour être associé à un lavabo placé dans les hospitaux afin de permettre au personnel hospitalier et notamment aux chirurgiens et aux personnels des salles d'opération de se laver les mains avant et après une opération.

Le distributeur 1 est constitué par un boîtier 2 ici de forme sensiblement parallélépipédique. Ce boîtier 2 venu des moulages est réalisé par des techniques classiques de plasturgie en matière de synthèse dure. Bien entendu, il pourrait être réalisé dans tout autre type de matière telle que par exemple en inox ou en toute matière facilement nettoyable, et il pourrait être de tout autre type de forme polygonale telle que par exemple carrée.

Le boîtier 2 présente un fond 3 et une face avant 4 ouverte. Il est délimité par deux parois latérales 5 et 6 et deux parois transversales 7 et 8. Ces parois 5, 6, 7, 8 présentent un rebord faisant saillie perpendiculairement qui est destiné à permettre la mise en place du distributeur 1 dans un logement correspondant ménagé par exemple sur un mur ou dans un rebord du lavabo.

Le boîtier 1 présente des moyens 10 pour faciliter la mise en place des contenants à l'intérieur du distributeur 1.

Ces moyens 10 sont ici constitués par un plan incliné 11 ménagé à la base 13 du boîtier 2 ainsi que par une rampe guide inclinée 14 placée sur chaque face latérale 5 et 6 du boîtier 2 de part et d'autre d'un plan médian vertical.

Le boîtier 2 comporte également des moyens 15 pour maintenir un contenant à l'intérieur du distributeur dans une position déterminée en fonction de sa conformation.

Ces moyens 15 sont délimités par trois zones d'appui 16, 17, 18 superposées et distinctes destinées à permettre la mise en place et le maintien efficace de contenants qui présentent des formes diverses comme il sera décrit ultérieurement.

La zone 16 est constituée par un plan horizontal 19 s'étendant en prolongement du plan incliné 11 perpendiculairement au fond 3 et, d'autre part par un rebord 20 faisant saillie perpendiculaire au fond 3 et parallèlement au plan 11. Ce rebord 20 est ici sensiblement hémi-circulaire en présentant un rayon de courbure qui est disposé sensiblement sur l'axe médian longitudinal du boîtier.

Dans la forme des réalisations décrites ci-dessus, le rebord 20 est constitué par deux quarts de cercle 21, 22 qui se rejoignent tangentiellement au niveau de l'axe médian horizontal du boîtier 2.

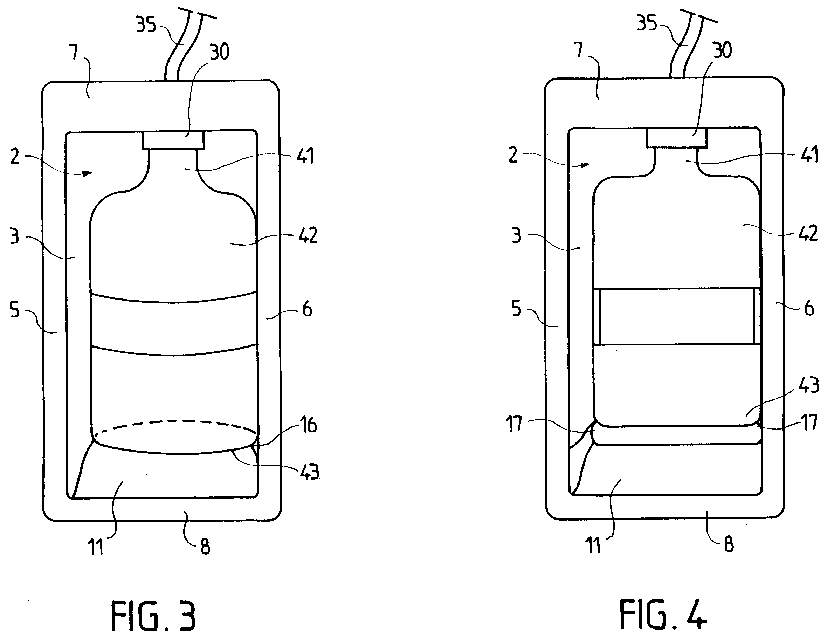

Ainsi, en combinaison avec le plan 19 et avec la rampe guide inclinée 14, ainsi qu'avec le fond 3 du boîtier, il délimite un logement qui permet de servir de maintien et de retenue à un contenant de forme circulaire comme il est plus particulièrement illustré à la figure 3.

La zone d'appui 17 est constituée d'une part par un méplat horizontal 23 délimitant le bord supérieur du rebord 20 et faisant saillie perpendiculairement au fond 3 du boîtier 1 et d'autre part par le fond 3 du boîtier 2 lui-même. En combinaison avec la rampe 14 et avec le fond du boîtier, il délimite un logement destiné à recevoir un contenant qui présente une base polygonale de préférence carrée ou rectangulaire comme il est plus particulièrement illustré à la figure 4.

La zone d'appui 18 est constituée par deux rebords 24 disposées symétriquement de part et d'autre d'un plan médian vertical. Ces deux rebords 24 placés sur chaque paroi latérale constituent le prolongement perpendiculaire de chaque rail guide 14.

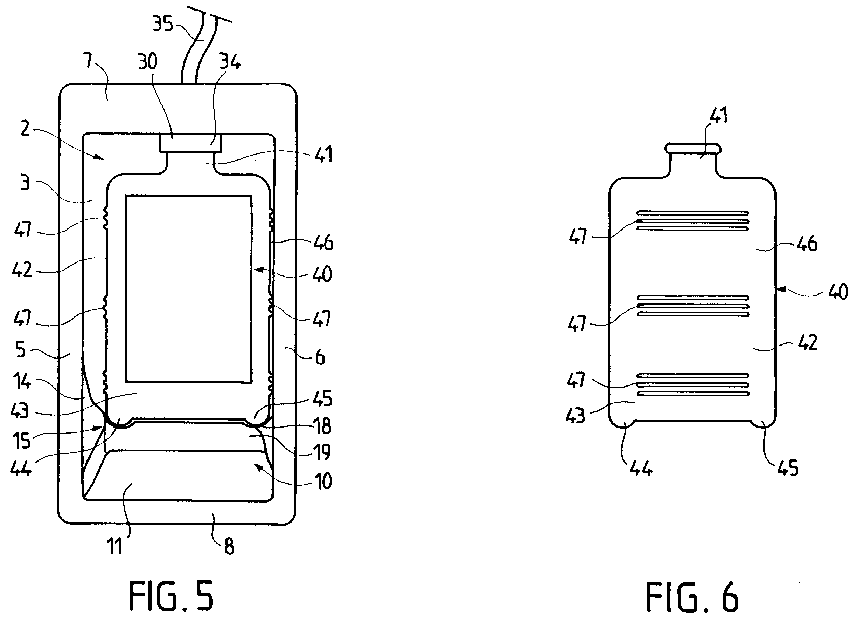

Avec le fond 3 du boîtier 2, il délimite un logement destiné à recevoir un contenant tel que celui qui est plus particulièrement représenté à la figure 6 dont la base est polygonale et de préférence carrée ou rectangulaire.

Cette délimitation du boîtier permet ainsi au distributeur de recevoir des contenants de formes et de dimensions diverses, ce qui favorise son utilisation polyvalente.

La partie supérieure du boîtier 2 comporte un logement 30 de forme polygonale destiné à recevoir un goulot du contenant.

Pour faciliter l'introduction de ce goulot, l'une des faces 31 de ce logement 30 présente une paroi inclinée 32 destinée à jouer le rôle d'une rampe guide.

Le logement 30 débouche dans un orifice circulaire 34 à l'intérieur duquel est monté un goulot 41 du contenant 40 lorsque celui-ci est placé à l'intérieur du dispositif comme il est plus particulièrement illustré aux figures 3 à 6.

Un tuyau flexible 35 traverse l'orifice et débouche à l'intérieur du boîtier en s'étendant sensiblement sur toute la longueur de celui-ci. Ce tuyau flexible et souple 35 qui est relié à des organes de commande permet de mettre en communication le liquide ou le produit contenu à l'intérieur des contenants avec un orifice tel qu'un robinet ou tout autre dispositif d'ouverture.

Il faut également noter que ce tuyau flexible 35 joue également le rôle d'un ressort élastique qui favorise le maintien du contenant dans une position déterminée à l'intérieur du distributeur en combinaison avec les zones d'appui 16, 17, 18.

Bien entendu, pour s'adapter à des contenants de dimensions diverses, le tuyau 35 peut présenter une longueur qui peut être variable

Si l'on se réfère plus particulièrement à la figure 6, on voit un contenant 40 qui présente un goulot 41, un fût 42 et une base 43 qui repose sur deux pieds 44 et 45 disposés de part et d'autre d'un plan médian vertical. Ce contenant 40, comme ceux utilisés dans cette application, est réalisé de préférence dans un matériau de synthèse opaque et teinté afin d'éviter la pénétration des rayons lumineux en raison notamment de la présence d'iode dans les produits disposés à l'intérieur de ce contenant 40.

Pour faciliter le maintien dans le distributeur de ce contenant 40, chaque face latérale 46 présente une répartition d'ondulations 47 venues de moulages en faisant saillie perpendiculairement. Ces ondulations 47 coopèrent en position montée avec la paroi latérale 5 et 6 du boîtier 1 afin d'assurer un maintien efficace de ce contenant dans le boîtier 2 du distributeur.

Selon l'invention, la mise en place de contenants quelles que soient leur forme et leurs dimensions à l'intérieur du distributeur 1 est aisée puisqu'il suffit d'introduire la base 43 du contenant 40 sur le plan incliné 11. Dans cette position, il suffit ensuite de placer le tuyau flexible 35 à l'intérieur du contenant 40 puis d'exercer une force pour entrainer, en translation le contenant à l'intérieur du boîtier 2 jusqu'à une position de butée dans lequelle le goulot 41 du contenant est placé dans le logement 30.

En fonction de sa forme, ce contenant 40 prendra facilement et rapidement sa place à l'intérieur du boîtier 2 en étant maintenu dans l'une des zones d'appui 16, 17, 18.

C'est ainsi, comme plus particulièrement illustré à la figure 3, si le contenant 40 est un contenant de forme arrondie, sa base 43 est maintenue par la zone 17.

De même, comme illustré à la figure 4, si le contenant 40 est de forme polygonale et de préférence carrée ou rectangulaire, sa base est maintenue par la zone 16.

Enfin, dans le cas d'un contenant 40 plus spécifique tel que celui qui est représenté à la figure 6, sa base 43 est maintenue par la zone d'appui 18.

Il faut également noter que le retrait d'un contenant du distributeur pour le remplacer par un autre contenant est également facile. Pour ce faire, il suffit d'exercer une pression sur le goulot 41 du contenant 40 afin de sortir ce goulot 41 du logement 30. Sous l'action de cette pression, le goulot 30 glisse sur la rampe guide 31 ménagées dans le logement 30. La base 41 quitte également la zone d'appui 16, 17 ou 18 sur laquelle elle est maintenue et le contenant 40 sort du fond 3 du distributeur 2. Il suffit ensuite d'ôter le tuyau flexible 35 du contenant 40 pour le sortir complètement du distributeur.

Comme il va de soi, l'invention n'est pas limitée à la seule forme de réalisation qui a été décrite ci-dessus mais elle en embrase au contraire toutes les formes de réalisation.

## Revendications

1. Distributeur polyvalent destiné notamment à la distribution de produits de désinfection et/ou de nettoyage sous forme liquide, pâteuse ou pulvérulente placé à l'intérieur de contenants de forme variée, caractérisé en ce qu'il comprend un boîtier (2) qui présente une face (4) avant ouverte destinée à recevoir au moins un contenant et en ce qu'il comporte :
- des moyens (10) pour faciliter la mise en place du contenant à l'intérieur du boîtier,
- des moyens (15) pour maintenir le contenant dans une position déterminée à l'intérieur du boîter (2) en fonction de sa conformation,
- des moyens (35) souples et flexibles pour distribuer le liquide depuis l'intérieur du contenant vers l'extérieur du distributeur.

2. Distributeur selon la revendication 1, caractérisé en ce que les moyens (10) pour faciliter la mise en place du contenant sont constitués par d'une part un plan guide incliné (11) ménagé à la base du boîtier (2), et d'autre part, par une rampe guide (14) placée sur les parois latérales (5, 6) du boîtier (2) de part et d'autre d'un plan médian vertical.

3. Distributeur selon la revendication 1, caractérisé en ce que les moyens (15) pour maintenir les contenants délimitent trois zones d'appui (16, 17, 18) distinctes et indépendantes.

4. Distributeur selon la revendication 3, caractérisé en ce que la zone d'appui (16) est constituée d'une part par un plan horizontal (19) s'étendant en prolongement du plan incliné (14) perpendiculairement au fond (3) du boîtier (2) et, d'autre part, par un rebord (20) faisant saillie perpendiculairement au fond (3), en présentant une forme sensiblement hémi-circulaire.

5. Distributeur selon la revendication 3, caractérisé en ce que la zone d'appui (17) est constituée par un méplat horizontal (23) délimitant le bord supérieur du rebord (20) en faisant saillie perpendiculairement au fond (3) du boîtier (2).

6. Distributeur selon la revendication 3, caractérisé en ce que la troisième zone d'appui (18) est constituée par deux rebords (24) disposés symétriquement de part et d'autre d'un plan médian vertical perpendiculairement au fond (3) du boîtier (2) dans le prolongement perpendiculaire de chaque rail guide (24).

7. Distributeur selon la revendication 1, caractérisé en ce que la partie supérieure du boîtier (2) présente un logement (30) de forme polygonale.

8. Distributeur selon la revendication 7, caractérisé en ce que l'une des faces (31) du logement (30) présente une paroi inclinée (32).

9. Distributeur selon la revendication 1, caractérisé en ce qu'il comporte un tuyau flexible (35) et souple qui traverse le logement (10) et s'étend au moins sur tout le fond (3) du boîtier (2).

10. Distributeur selon la revendication 1, caractérisé en ce que les parois latérales (46) du contenant (10) comportent une répartition régulière d'ondulations (47) faisant saillie perpendiculairement.

## Claims

1. Multi-purpose dispenser designed, in particular, for dispensing disinfecting and/or cleaning products in liquid, paste or powder form placed inside containers of various shapes, characterized in that it includes a housing (2) having an open front face (4) designed to receive at least one container, and in that it comprises:
- (10) for facilitating the installation of the container inside the housing;
- means (15) for holding the container in a given position inside the housing (2) as a function of its conformation;
- supple and flexible means (35) for dispensing the liquid from the inside of the container out of the dispenser.

2. Dispenser according to claim 1, characterized in that the means (10) for facilitating the installation of the container are constituted, on one hand, by an inclined guide plane (11) provided at the base of the housing (2) and, on the other hand, by a guide ramp (14) placed on the side walls (5, 6) of the housing (2) on either side of a vertical median plane.

3. Dispenser acording to claim 1, characterized in that the means (15) for holding the containers delimit three separate, independent bearing areas (16, 17, 18).

4. Dispenser according to claim 3, characterized in that bearing area (16) is formed, on one hand, by a horizontal plane (19) extending in continuation of the inclined plane (14) perpendicular to the rear (3) of the housing (2) and, on the other hand, by an edge member (20) projecting perpendicular to the rear (3) and having a substantially semi-circular shape.

5. Dispenser according to claim 3, characterized in that bearing area (17) is formed by a horizontal flat portion (23) delimiting the upper edge of the edge member (20) and projecting perpendicular to the rear (3) of the housing (2).

6. Dispenser according to claim 3, characterized in that the third bearing area (18) is formed by two edge members (24) disposed symetrically on either side of a vertical median plane perpendicular to the rear (3) of the housing (2) in perpendicular continuation of each guide rail (24).

7. Dispenser according to claim 1, characterized in that the upper portion of the housing (2) has a polygonal recess (30).

8. Dispenser accoding to claim 7, characterized in that one of the faces (31) of the recess (30) has an inclined wall (32).

9. Dispenser according to claim 1, characterized in that it comprises a flexible, supple hose (35) that passes through the housing (10) and extends at least over all of the rear (3) of the housing (2).

10. Dispenser according to claim 1, characterized in that the side walls (46) of the container (10) comprise a regular distribution of perpendicularly projecting corrugations (47).

## Patentansprüche

1. Mehrzweck-Spender, nämlich zum Spenden von innerhalb von Behältern unterschiedlicher Form untergebrachten Desinfektions- und Reinigungsmitteln in flüssiger, dickflüssiger oder Pulverform, dadurch gekennzeichnet, daß er ein Gehäuse (2) umfaßt, das eine offene Vorderseite (4) zum Aufnehmen wenigstens eines Behälters aufweist, und daß er:
- Mittel (10) zum Erleichtern des Anbringens des Behälters in das Innere des Gehäuses,
- Mittel (15), um den Behälter je nach dessen Form in einer bestimmten Lage innerhalb des Gehäuses (2) zu halten,
- biegsame und flexible Mittel (35) zum Spenden der Flüssigkeit vom Inneren des Behälters zur Außenseite des Spenders,
umfaßt.

2. Spender nach Anspruch 1, dadurch gekennzeichnet, daß die Mittel (10) zum Erleichtern des Anbringens des Behälters zum einen aus einer an der Basis des Gehäuses (2) vorgesehenen, schiefen Fürungsebene (11) und zum anderen aus einer beidseitig einer senkrechten Mittelebene an den Seitenwänden (5, 6) des Gehäuses (2) angebrachten Führungsrampe (14) bestehen.

3. Spender nach Anspruch 1, dadurch gekennzeichnet, daß die Mittel (15) zum Halten der Behälter drei unterschiedliche und unabhängige Abstützbereiche (16, 17, 18) begrenzen.

4. Spender nach Anspruch 3, dadurch gekennzeichnet, daß der Abstützbereich (16) zum einen aus einer sich in der Verlängerung der schiefen Ebene (14) senkrecht zum Boden (3) des Gehäuses (2) erstreckenden waagerechten Ebene (19) und zum anderen aus einem senkrecht zum Boden (3) hervorstehenden Rand (20) besteht, wobei dieser letzte eine im wesentlichen halbkreisförmige Gestalt aufweist.

5. Spender nach Anspruch 3, dadurch gekennzeichnet, daß der Abstützbereich (17) aus einer waagerechten Fläche (23) besteht, die die obere Kante des Randes (20) begrenzt, wobei sie senkrecht zum Boden (3) des Gehäuses (2) hervorsteht.

6. Spender nach Anspruch 3, dadurch gekennzeichnet, daß der dritte Abstützbereich (18) aus zwei beidseitig einer senkrechten Mittelebene symmetrisch, senkrecht zum Boden (3) des Gehäuses (2), in der senkrechten Verlängerung jeder Führungsschiene (24) angeordneten Rändern (24) besteht.

7. Spender nach Anspruch 1, dadurch gekennzeichnet, daß der obere Teil des Gehäuses (2) einen vieleckförmigen Hohlraum (30) aufweist.

8. Spender nach Anspruch 7, dadurch gekennzeichnet, daß eine Fläche (31) des Hohlraums (30) eine geneigte Wand (32) aufweist.

9. Spender nach Anspruch 1, dadurch gekennzeichnet, daß er einen flexiblen und biegsamen Schlauch (35) aufweist, der den Hohlraum (10) durchquert und sich wenigstens ûber den ganzen Boden (3) des Gehäuses (2) erstreckt.

10. Spender nach Anspruch 1, dadurch gekennzeichnet, daß die Seitenwände (46) des Behälters (10) eine regelmäßige Verteilung von senkrecht hervorstehenden Wellen (47) umfassen.
